# FASCICULE DE BREVET EUROPEEN

(11) **EP 3 888 797 B1**
(45) Date de publication et mention de la délivrance du brevet: **27.12.2023**
(21) Numéro de dépôt: 21165555.0
(22) Date de dépôt: 29.03.2021
(51) Int. Cl.: B05B 11/02

(54) **DISPOSITIF DE DISTRIBUTION DE PRODUIT FLUIDE**
VORRICHTUNG ZUM VERTEILEN VON FLÜSSIGEN PRODUKTEN
DEVICE FOR DISPENSING A LIQUID PRODUCT

(30) Priorité: 30.03.2020 FR 2003151
(43) Date de publication de la demande: 06.10.2021
(73) Titulaire: APTAR France SAS, 27110 Le Neubourg (FR)
(72) Inventeur: DESCHAMPS, Olivier, 27610 ROMILLY SUR ANDELLE (FR); BERTHELOT, Jonathan, 27100 LE VAUDREUIL (FR)
(74) Mandataire: CAPRI

(56) Documents cités:
- WO-A1-03/020436
- DE-C1- 19 545 226
- FR-A1- 2 793 707
- US-A- 4 475 905
- US-A1- 2011 033 224

## Description

La présente invention concerne un dispositif de distribution de produit fluide, et plus particulièrement un dispositif de distribution de produit fluide destiné à distribuer un nombre limité de doses, tel qu'un unidose ou un bidose.

Les dispositifs de type unidose ou bidose sont bien connus dans l'état de la technique et trouvent une partie de leurs applications dans le domaine des distributeurs pharmaceutiques à utilisation nasale, dans lesquelles une dose de produit doit être distribuée dans une narine, respectivement dans chacune des deux narines dans le cas d'un bidose.

Le document FR2761281 divulgue un tel dispositif bidose. Pour utiliser ce dispositif, l'utilisateur place l'orifice de distribution dans sa narine et exerce une pression axiale sur l'élément d'actionnement du dispositif. Cette pression axiale doit être suffisamment élevée pour surmonter des moyens d'accumulation d'énergie afin de garantir la distribution de la totalité de chacune des deux doses. Lors de l'application de cette force axiale relativement importante, il est difficile de contrôler précisément la position de l'extrémité du dispositif comportant l'ouverture de distribution dans la narine, et il peut arriver que le bout de la tête du dispositif vienne buter au fond de la narine, ce qui peut être gênant et/ou douloureux pour l'utilisateur. D'autre part, du fait que ce dispositif nécessite une force minimale prédéterminée pour pouvoir être actionné, il peut être difficile à utiliser pour des personnes âgées ou peu mobiles qui peuvent avoir des difficultés à exercer cette force avec le dispositif placé dans la narine. Le document WO 03/020436 A1 montre un dispositif de distribution de produit fluide pour les ingrédients pharmaceutiques actifs.

La présente invention a pour but de fournir un dispositif de distribution de produit fluide qui ne présente pas les inconvénients susmentionnés.

En particulier, la présente invention a pour but de fournir un dispositif de distribution de produit fluide, tel qu'un unidose ou un bidose, qui soit simple et fiable à utiliser, et dans lequel la distribution du produit est indépendante de l'effort d'actionnement de l'utilisateur.

La présente invention a également pour but de fournir un tel dispositif de distribution qui garantisse la distribution de la totalité d'une dose à chaque actionnement.

La présente invention a aussi pour but de fournir un tel dispositif de distribution qui évite les risques de blessures lors de l'actionnement.

La présente invention a également pour but de fournir un tel dispositif qui facilite l'assemblage et le remplissage, et qui notamment permette d'utiliser un réservoir prérempli, de sorte que l'assemblage final du dispositif n'est pas nécessairement à réaliser dans un environnement stérile.

La présente invention a encore pour but de fournir un tel dispositif de distribution qui soit simple et peu coûteux à fabriquer et à assembler.

La présente invention a donc pour objet un dispositif de distribution de produit fluide tel que décrit dans la revendication 1. Des modes de réalisation avantageux sont décrits dans les revendications dépendantes.

Ces avantages et caractéristiques et d'autres de la présente invention apparaîtront au cours de la description détaillée suivante de plusieurs variantes de réalisation, données à titre d'exemples non limitatifs, en regard des dessins joints, sur lesquels :
la figure 1 est une vue schématique partielle en section transversale d'un dispositif de distribution de produit fluide du type unidose selon un mode de réalisation de la présente invention, avant distribution de la dose,
la figure 2 est une vue similaire à celle de la figure 1, après distribution de la dose,
la figure 3 est une représentation schématique de dessus du dispositif de la figure 1,
la figure 4 est une représentation schématique de dessus du dispositif de la figure 2,
la figure 5 est une vue schématique éclatée en section transversale d'un dispositif de distribution de produit fluide du type bidose selon un autre mode de réalisation, avant assemblage,
les figures 6 à 8 sont des représentations schématiques partielles du dispositif de la figure 5, respectivement avant actionnement, après distribution de la première dose, et après distribution de la seconde dose, et
la figure 9 à 13 sont des vues schématiques partielles du dispositif de la figure 5, respectivement avant actionnement, avant et après distribution de la première dose, et avant et après distribution de la seconde dose.

Dans la description ci-après, les termes "axial" et "radial" se réfèrent à l'axe central longitudinal du dispositif.

La présente invention va être faite en référence à deux variantes de réalisations représentées sur les dessins et qui concernent des dispositifs de distribution de produit fluide du type unidose ou bidose, c'est-à-dire comportant une ou deux doses de produit à distribuer, le produit étant destiné à être distribué dans le nez de l'utilisateur. Il est entendu que la présente invention s'applique également à des dispositifs du type multi-doses comportant plus que deux doses, par exemple trois ou quatre. De plus, la présente invention n'est pas limitée aux distributeurs de type nasal.

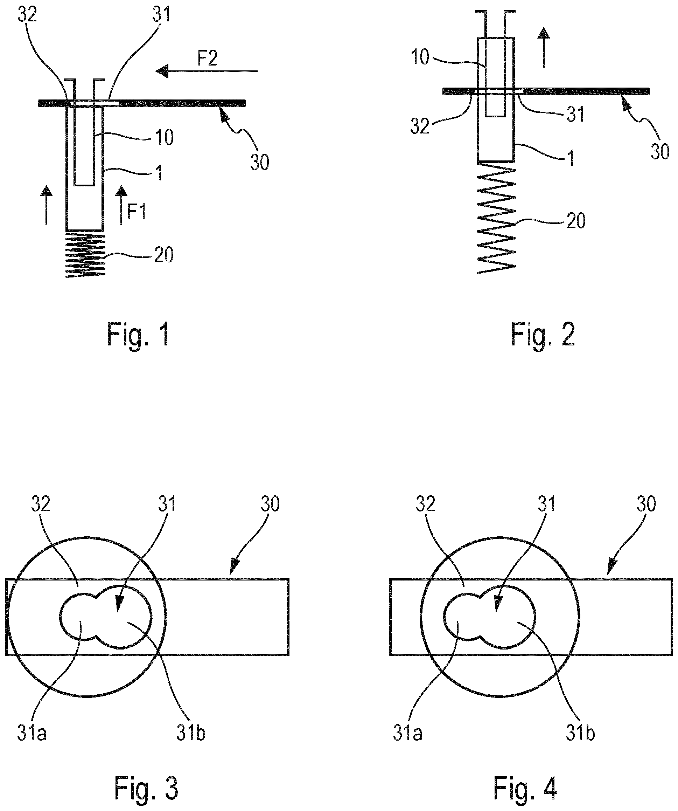

En référence aux figures 1 à 4, il est représenté de manière très schématique un mode de réalisation de la présente invention. Dans ce mode de réalisation, le dispositif comporte un corps 1 recevant un réservoir 10, de préférence formé d'un tube. Ce réservoir 10 peut être rempli avec le produit à distribuer puis obturé de manière étanche par un bouchon approprié (non représenté), ledit réservoir prérempli 10 pouvant ensuite être adapté dans le corps 1.

Le corps 1 est déplaçable axialement, dans le sens de la flèche F1 sur la figure 1, entre une position de repos et une position actionnée, et coopère avec un élément élastique 20, tel qu'un ressort, qui sollicite ledit corps 1 vers sa position actionnée.

Des moyens de blocage 30, déplaçables entre une position de blocage et une position de non blocage, retiennent le corps 1 en position de repos. Ces moyens de blocage 30 sont déplaçables manuellement par l'utilisateur entre une position de blocage et une position de non blocage, ledit déplacement étant réalisé dans une direction différente de la direction de déplacement axiale dudit corps 1 entre ses positions de repos et actionnée.

Dans l'exemple des figures 1 à 4, les moyens de blocage comportent une plaque 30 déplaçable en translation radialement par rapport audit corps 1, dans le sens de la flèche F2 sur la figure 1.

Cette plaque 30 comporte une ouverture 31, recevant le corps 1. En l'absence de corps, l'ouverture 31 pourrait recevoir directement le réservoir.

L'ouverture 31 comporte une première partie d'ouverture 31a de plus petite dimension et une seconde partie d'ouverture 31b de plus grande dimension. Comme visible sur les figures 3 et 4, les première et seconde parties d'ouverture 31a, 31b sont interconnectées.

Avantageusement, la première partie d'ouverture 31a peut être disposée d'un côté radial de la plaque 30 et la seconde partie d'ouverture 31b peut être disposée du côté radial opposé de ladite plaque 30.

Le bord 32 de la première partie d'ouverture 31a coopère en position de blocage avec le corps 1 pour le retenir en position de repos.

Lorsque la plaque 30 est déplacée latéralement par l'utilisateur, la première partie d'ouverture 31a se décale du corps 1, et lorsque la seconde partie d'ouverture 31b se retrouve face au corps 1, celui-ci n'est plus retenu par le bord 32 de la première partie d'ouverture 31a, et le corps 1, ensemble avec le réservoir 10, se déplace alors axialement vers la position actionnée sous l'effet du ressort 20 comprimé.

Ce déplacement axial provoque la distribution de la dose de produit fluide contenu dans le réservoir 10 d'une quelconque manière connue. Les documents EP0546607 et EP0311863 décrivent des dispositifs du type unidose dans lesquels la dose de produit fluide est, distribuée lors du déplacement axial du réservoir.

Dans l'exemple des figures 1 à 4, les première et seconde parties d'ouverture 31a, 31b sont de forme circulaires interconnectées. En variante, on pourrait avoir une forme globale oblongue s'élargissant de manière régulière en direction de la seconde partie d'ouverture 31b. D'autres formes sont aussi envisageables, à la condition qu'elles soient interconnectées et qu'elles réalisent un blocage du corps 1 en position de blocage des moyens de blocage et une libération dudit corps en position de non blocage.

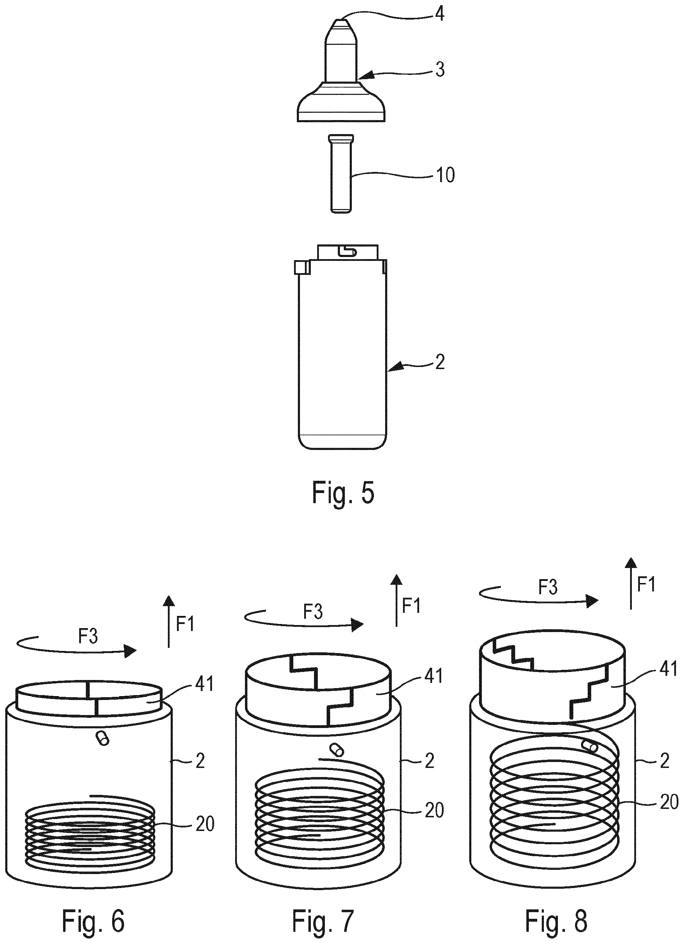

En référence aux figures 5 à 13, il est représenté de manière très schématique un second mode de réalisation.

Un réservoir 10, contenant une ou plusieurs doses de produit fluide à distribuer, et monté dans corps de base 2, sur lequel est assemblé une tête de distribution 3 pourvue d'un orifice de distribution 4.

Le réservoir 10 est déplaçable axialement par rapport au corps de base 2 et à la tête de distribution 3 lors de l'actionnement, sous l'effet d'un ressort 20 comprimé disposé dans le corps de base 2.

Dans l'exemple représenté, le réservoir 10 est fixé dans un corps mobile 41, qui est déplaçable axialement dans le sens de la flèche F1 et en rotation dans le sens de la flèche F3 sur les figures 6 à 8. Le ressort comprimé 20 sollicite le corps mobile 41 axialement vers le haut, et des moyens de blocage 51, 53, 55 sont prévus pour empêcher ledit déplacement axial.

Les figures 6 à 8 illustrent la cinématique des moyens de blocage: à chaque rotation du corps mobile 41 dans le sens de la flèche F3, une course axiale dans le sens de la flèche F1 est réalisée.

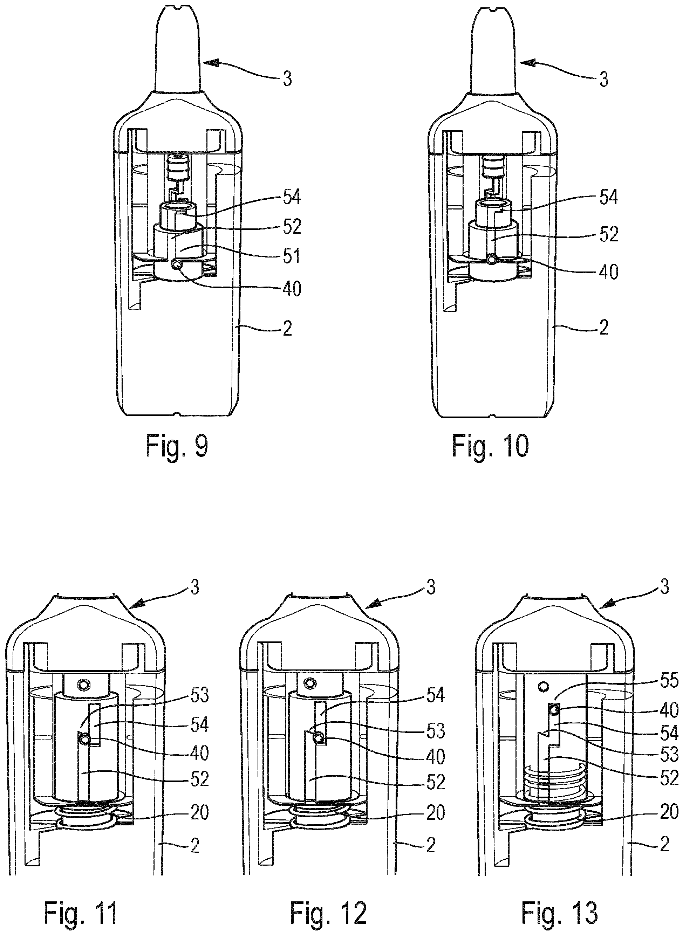

Les figures 9 à 13 illustrent un exemple de réalisation, dans lequel le corps mobile 41 comporte un ergot 40 coopérant avec des rainures axiales 52, 54 décalées latéralement, une rainure axiale respective correspondant à une dose distribuée respective. Dans cet exemple, qui comporte deux rainures axiales 52, 54, le dispositif est donc du type bidose.

Avant le premier actionnement, l'ergot 40 est décalé par rapport à la première rainure 52, et est bloqué axialement par une première paroi 51 de ladite première rainure 52, comme visible sur la figure 9.

Une rotation du corps mobile déplace l'ergot 40 en face de ladite première rainure 52, de sorte que le ressort comprimé 20 déplace ledit corps mobile et donc ledit réservoir 10 axialement vers le haut pour distribuer la première dose, comme visible sur la figure 10. La rotation dudit corps mobile peut être réalisée dans un sens quelconque, soit dans le sens de la flèche F3, comme représenté schématiquement sur la figure 6, soit dans un sens inverse, comme illustré sur les figures 9 et 10.

Lorsque l'ergot 40 atteint le haut de la première rainure 52, il bute contre une seconde paroi 53 qui le bloque à nouveau axialement, comme visible sur la figure 11.

Une nouvelle rotation du corps mobile déplace l'ergot 40 en face de la seconde rainure 54, comme visible sur la figure 12, de sorte que le ressort comprimé 20 déplace ledit corps mobile et donc ledit réservoir 10 axialement vers le haut dans ladite seconde rainure 54 pour distribuer la seconde dose.

Lorsque l'ergot 40 atteint le haut de la seconde rainure 54, il bute contre une troisième paroi 55 qui le bloque à nouveau axialement, comme visible sur la figure 13.

Dans le cas d'un bidose, il s'agit là de la fin de la course d'actionnement du dispositif, et l'ergot 40 n'est alors plus déplaçable, ni axialement, ni en rotation.

La présente invention fournit donc un dispositif de distribution, notamment du type unidose ou bidose, qui assure notamment les fonctions suivantes :
- il facilite la distribution du produit par un actionnement latéral, la distribution du produit étant indépendante de l'effort de l'utilisateur, puisque c'est un ressort qui assure cette distribution ; en particulier, le ressort garantit les caractéristiques constantes du spray ;
- il garantit l'étanchéité du produit contenu dans le réservoir avant, pendant et après l'actionnement du dispositif ;
- il permet d'utiliser un réservoir prérempli, de sorte que l'assemblage du dispositif peut être réalisé dans des conditions non stériles ;
- il assure une sécurité d'utilisation en permettant la distribution des doses dans n'importe quelle position du dispositif et en empêchant un actionnement accidentel de la seconde dose avant distribution de la première dose ;
- il limite les risques de contamination du produit en mettant celui-ci en contact avec seulement deux matériaux, à savoir le matériau du réservoir, en général du verre, et le matériau du piston-bouchon, en général un matériau élastomère inerte vis-à-vis du produit.

Bien entendu, comme déjà précisé en début de description, la présente invention n'est ni limitée aux dispositifs de type unidose ou bidose, ni aux dispositifs de type nasal. On pourrait par exemple envisager un tel dispositif pour distribuer trois ou quatre doses, par exemple dans les yeux, les oreilles ou la bouche de l'utilisateur.

## Revendications

1. Dispositif de distribution de produit fluide comportant un réservoir (10) contenant une ou plusieurs doses de produit fluide, un élément élastique (20), tel qu'un ressort, pour solliciter axialement ledit réservoir (10) entre une position de repos et au moins une position actionnée, et des moyens de blocage (30; 51, 53, 55) pour empêcher le déplacement axial dudit réservoir (10), lesdits moyens de blocage étant déplaçables manuellement dans une direction différente de ladite direction de déplacement axial du réservoir (10) pour permettre le déplacement axial dudit réservoir (10), **caractérisé en ce que** lesdits moyens de blocage comportent une plaque (30) pourvue d'une ouverture (31) comportant une première partie d'ouverture (31a) de plus petite dimension radiale et une seconde partie d'ouverture (31b) de plus grande dimension radiale, lesdites première et seconde parties d'ouverture (31a, 31b) étant interconnectées, ladite plaque (30) étant déplaçable radialement entre une position de blocage, dans laquelle ladite première partie d'ouverture (31a) coopère avec ledit réservoir (10) pour le bloquer axialement, et une position de non blocage, dans laquelle ladite seconde partie d'ouverture (31b) coopère avec ledit réservoir (10) pour permettre le déplacement axial dudit réservoir (10) sous l'effet dudit élément élastique (20).

2. Dispositif selon la revendication 1, dans lequel ledit réservoir (10) est fixé dans un corps (1), ladite ouverture (31) de ladite plaque (30) recevant ledit corps (1) et les bords de ladite ouverture (31) coopérant avec ledit corps (1).

3. Dispositif selon la revendication 1 ou 2, dans lequel lesdites première et seconde parties d'ouverture (31a, 31b) sont de forme circulaires interconnectées.

4. Dispositif selon l'une quelconque des revendications précédentes, dans lequel ledit réservoir (10) contient une dose de produit fluide distribuée en un seul actionnement.

5. Dispositif selon l'une quelconque des revendications 1 à 3, dans lequel ledit réservoir (10) contient deux doses de produit fluide distribuée en deux actionnements successifs.

## Patentansprüche

1. Vorrichtung zur Abgabe eines Fluidprodukts aufweisend einen Vorratsbehälter (10), der eine oder mehrere Fluidproduktdosen enthält, ein elastisches Element (20) wie etwa eine Feder zur axialen Vorspannung des Vorratsbehälters (10) zwischen einer Ruheposition und wenigstens einer betätigten Position und Blockiermittel (30; 51, 53, 55) zur Verhinderung der axialen Bewegung des Vorratsbehälters (10), wobei die Blockiermittel manuell in einer Richtung bewegbar sind, die sich von der Richtung der axialen Bewegung des Vorratsbehälters (10) unterscheidet, um die axiale Bewegung des Vorratsbehälters (10) zu ermöglichen, **dadurch gekennzeichnet, dass** die Blockiermittel eine Platte (30) aufweisen, die mit einer Öffnung (31) versehen ist, die einen ersten Öffnungsabschnitt (31a) mit kleinerer Radialabmessung und einen zweiten Öffnungsabschnitt (31b) mit größerer Radialabmessung aufweist, wobei der erste und zweite Öffnungsabschnitt (31a, 31b) miteinander verbunden sind, wobei die Platte (30) radial zwischen einer Blockierposition, in welcher der erste Öffnungsabschnitt (31a) mit dem Vorratsbehälter (10) zusammenwirkt, um ihn axial zu blockieren, und einer Nichtblockierposition, in welcher der zweite Öffnungsabschnitt (31b) mit dem Vorratsbehälter (10) zusammenwirkt, um die axiale Bewegung des Vorratsbehälters (10) unter der Einwirkung des elastischen Elements (20) zu ermöglichen, bewegbar ist.

2. Vorrichtung nach Anspruch 1, wobei der Vorratsbehälter (10) in einem Körper (1) befestigt ist, wobei die Öffnung (31) der Platte (30) den Körper (1) aufnimmt und die Ränder der Öffnung (31) mit dem Körper (1) zusammenwirken.

3. Vorrichtung nach Anspruch 1 oder 2, wobei der erste und zweite Öffnungsabschnitt (31a, 31b) miteinander verbundene Kreisformen aufweisen.

4. Vorrichtung nach einem der vorhergehenden Ansprüche, wobei der Vorratsbehälter (10) eine Fluidproduktdosis enthält, die mit einer einzelnen Betätigung abgegeben wird.

5. Vorrichtung nach einem der Ansprüche 1 bis 3, wobei der Vorratsbehälter (10) zwei Fluidproduktdosen enthält, die mit zwei aufeinanderfolgenden Betätigungen abgegeben werden.

## Claims

1. A fluid dispenser device comprising: a reservoir (10) containing one or more doses of fluid; a resilient element (20), such as a spring, for urging said reservoir (10) to move axially between a rest position and at least one actuated position; and blocking means (30; 51, 53, 55) for preventing said reservoir (10) from moving axially; said blocking means being movable manually in a direction that is different from the direction of said axial movement of the reservoir (10) so as to allow said reservoir (10) to move axially; the device being **characterized in that** said blocking means comprise a plate (30) provided with an opening (31) comprising a first opening portion (31a) of smaller radial size, and a second opening portion (31b) of larger radial size, said first and second opening portions (31a, 31b) being interconnected, said plate (30) being movable radially between a blocking position in which said first opening portion (31a) cooperates with said reservoir (10) so as to block it axially, and a non-blocking position in which said second opening portion (31b) cooperates with said reservoir (10) so as to allow said reservoir (10) to move axially under the effect of said resilient element (20).

2. A device according to claim 1, wherein said reservoir (10) is fastened in a body (1), said opening (31) of said plate (30) receiving said body (1) and the edges of said opening (31) co-operating with said body (1).

3. A device according to claim 1 or claim 2, wherein said first and second opening portions (31a, 31b) are in the shape of interconnected circles.

4. A device according to any preceding claim, wherein said reservoir (10) contains a dose of fluid for dispensing in a single actuation.

5. A device according to any one of claims 1 to 3, wherein said reservoir (10) contains two doses of fluid for dispensing in two successive actuations.
